(19) Europäisches Patentamt
European Patent Office
Office européen des brevets

(11) Veröffentlichungsnummer : **0 084 853**
**B1**

(12) # EUROPÄISCHE PATENTSCHRIFT

(45) Veröffentlichungstag der Patentschrift : **12.11.86**

(51) Int. Cl.[4] : **C 07 D209/92**

(21) Anmeldenummer : **83100441.1**

(22) Anmeldetag : **19.01.83**

(54) **Verfahren zur Herstellung von 1,8-Naphtholactamverbindungen.**

(30) Priorität : **22.01.82 CH 407/82**

(43) Veröffentlichungstag der Anmeldung : **03.08.83 Patentblatt 83/31**

(45) Bekanntmachung des Hinweises auf die Patenterteilung : **12.11.86 Patentblatt 86/46**

(84) Benannte Vertragsstaaten : **CH DE FR GB LI**

(56) Entgegenhaltungen :
**EP-A- 0 017 621**
**CH-A- 521 976**
**ANNALEN DER CHEMIE, Band 655, 1962, VERLAG CHEMIE GMBH, Weinheim, DE JUSTUS LIEBIGS**
**Synthetic Methods of Organic Chemistry Band 29 (1975), Seite 201 (387)**
**Houben-Weyl Band VI/2, Seite 793-4**
**Houben-Weyl Band XI/2, Seite 539**

(73) Patentinhaber : **CIBA-GEIGY AG**
**Klybeckstrasse 141**
**CH-4002 Basel (CH)**

(72) Erfinder : **Begrich, Rainer, Dr.**
**Habsburgerstrasse 32**
**CH-4310 Rheinfelden (CH)**

(74) Vertreter : **Zumstein, Fritz jun., Dr. et al**
**Dr. F. Zumstein sen. Dr. E. Assmann Dr. R. Koenigsberger Dipl.-Ing. F. Klingseisen Dr. F. Zumstein jun.**
**Bräuhausstrasse 4**
**D-8000 München 2 (DE)**

**EP 0 084 853 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von 1,8-Naphtholactamverbindungen der Formel I

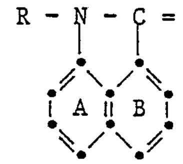

(I)

worin R Wasserstoff, ein gegebenenfalls durch $C_1$-$C_4$-Alkoxy, Halogen, Hydroxy, Cyano, Carboxy, N,N-Dialkyl($C_1$-$C_4$)-carbamoyl oder Phenyl substituierter $C_1$-$C_4$-Alkylrest, ein Cyclohexylrest oder ein gegebenenfalls durch Halogen, $C_1$-$C_4$-Alkyl, $C_1$-$C_4$-Alkoxy, Cyano, Carboxy, N,N-Dialkyl($C_1$-$C_4$)-carbamoyl- oder N,N-Dialkyl($C_1$-$C_4$)-sulfamoyl substituierter Phenylrest ist und die Benzolringe A und/oder B gegebenenfalls durch Halogen, Cyano, Nitro, $C_1$-$C_4$-Alkyl, $C_1$-$C_4$-Alkoxy, $C_1$-$C_4$-Alkylsulfonyl, Phenylsulfonyl, Alkyl-($C_1$-$C_4$)-carbonylamino, Benzoylamino, Carboxyl, $C_1$-$C_4$-Alkoxycarbonyl, Carbamoyl, N-Mono- oder N,N-Dialkyl-($C_1$-$C_4$)-carbamoyl, Sulfamoyl, N-Mono- oder N,N-Dialkyl-($C_1$-$C_4$)-sulfamoyl substituiert sind. Das neue Verfahren ist dadurch gekennzeichnet, dass man eine 1,8-Naphtholactonverbindung der Formel II

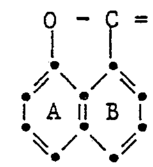

(II)

mit einem Amin der Formel III

$$NH_2—R \qquad (III)$$

gegebenenfalls in Gegenwart von Bisulfit umsetzt.

Der $C_1$-$C_4$-Alkylrest, bei es als Rest R oder als Substituent des Phenylrestes oder der Benzolringe A und/oder B, kann unverzweigt oder verzweigt sein. Es handelt sich beispielsweise um den Methyl-, Aethyl-, n-Propyl- oder tert.-Butylrest. Der Alkylrest R kann substituiert sein und zwar z. B. durch eine $C_1$-$C_4$-Alkoxygruppe, wie die Methoxy-, Aethoxy-, n-Propoxy-, n-Butoxy- oder sec.-Butoxygruppe. Als Substituenten kommen ferner Halogenatome, wie z. B. Fluor, Chlor oder Brom in Frage. Der Phenylrest R kann nicht nur durch $C_1$-$C_4$-Alkyl, sondern z. B. auch durch Halogen, wie Fluor, Chlor oder Brom oder einen verzweigten bzw. unverzweigten Alkoxyrest mit 1 bis 4 C-Atomen substituiert sein.

Die Ringe des Naphthalingerüstes A und B können, wie bereits erwähnt, ebenfalls durch einen $C_1$ bis $C_4$-Alkylrest substituiert sein. Als weitere Substituenten sind genannt Halogen, wie Fluor, Chlor oder Brom, ferner $C_1$-$C_4$-Alkoxy und zwar ein unverzweigter oder verzweigter Alkoxyrest, wie z. B. der Methoxy ; Aethoxy- oder n-Propoxyrest. Bei dem $C_1$-$C_4$-Alkylsulfonylrest handelt es sich z. B. um die Methylsulfonyl- oder Aethylsulfonylgruppe und bei der Alkyl ($C_1$-$C_4$)-carbonylaminogruppe in erster Linie um den Acetylaminorest. Als N,N-Dialkyl-($C_1$-$C_4$)-sulfamoylgruppe ist beispielsweise die N,N-Dibutylsulfamoylgruppe (Dibutylaminosulfonylgruppe) genannt.

Das neue Verfahren wird derart durchgeführt, dass man eine 1,8-Naphtholactonverbindung der Formel II vorzugsweise im wässrigen Medium mit einem Amin der Formel III, gegebenenfalls in Gegenwart eines Bisulfits umsetzt. Dabei kann man entweder die Verbindung der Formel II in Wasser suspendieren und das Amin oder die wässrige Lösung des Amins der Formel III zugeben, oder man legt das Amin der Formel III oder seine wässrige Lösung vor und gibt die Verbindung der Formel II eventuell als wässrige Suspension zu ; es ist aber auch möglich, dass man die Verbindung der Formel II mit dem Amin der Formel III mischt und anschliessend gegebenenfalls Wasser zufügt. Anstelle des Wassers kann als Reaktionsmedium auch ein niederer aliphatischer Alkohol, wie z. B. Methanol, verwendet werden.

Vorteilhaft wird die Reaktion in Gegenwart eines Bisulfits durchgeführt. Als Bisulfits kommen z. B. Alkali-, Ammonium- und Erdalkalibisulfite in Frage wie vor allem Natriumbisulfit oder auch Calciumbisulfit. Es ist aber auch möglich Gemische verschiedener Bisulfite der Reaktion zuzusetzen oder auch das Bisulfit-Salz des Amins der Formel III. Man setzt das Bisulfit vorzugsweise in Form einer wässrigen Lösung (z. B. 40 %ige wässrige Lösung) zu.

Die Umsetzung gemäss vorliegender Erfindung verläuft besonders gut (bezogen auf die Ausbeute an der Naphtholactamverbindung der Formel I), wenn man mit 2 bis 10 Aequivalent und insbesondere 2 bis 5 Aequivalenten des Amins der Formel III und in Gegenwart von Bisulfit arbeitet. Vorteilhaft setzt man 0,1 bis 5 Aequivalente und insbesondere 0,5 bis 2,5 Aequivalente des Bisulfits, bezogen auf das eingesetzte 1,8-Naphtholacton der Formel II, ein.

Die Durchführung der Reaktion erfolgt vorzugsweise in einem Autoklaven während ca. 1 bis 24 Stunden bei einer Temperatur von etwa 100 bis 200 °C. Bei einer Temperatur von 150 °C ist die Reaktion z. B. nach ca. 15 Stunden beendet. Anschliessend lässt man die Reaktionsmasse abkühlen und filtriert oder trennt die flüssigen Phasen. Man erhält dabei die 1,8-Naphtholactamverbindungen der Formel I in hoher Reinheit und mit einer Ausbeute von über 90 %, bezogen auf die eingesetzte Lactonverbindung.

Die Naphtholactonverbindungen der Formel II sind bekannt oder können nach bekannten Methoden hergestellt werden. Erfindungsgemäss werden beispielsweise die folgenden eingesetzt :

Naphtholacton-1,8 ;
4-Chlor-naphtholacton-1,8 ;
4-Brom-naphtholacton-1,8 ;
2,4-Dibrom-naphtholacton-1,8 ;
5-Cyan-naphtholacton-1,8 ;
4-Methylsulfonyl-naphtholacton-1,8 ;
4-Dibutylaminosulfonyl-naphtholacton-1,8 ;
5-Aethyloxycarbonyl-naphtholacton-1,8 ; und
5-N-Methyl-carbamoyl-naphtholacton-1,8 .

Bekannt oder nach bekannten Methoden herstellbar sind ebenfalls die Amine der Formel III ; die folgenden können beispielsweise im erfindungsgemässen Verfahren eingesetzt werden : Ammoniak, Methylamin, Aethylamin, n-Propylamin, Methoxyäthylamin, 2-Cyanäthylamin, 3-Hydroxypropylamin, Chlormethylamin, Methanolamin ; Anilin, Anisidin (o-, m-, p-), Chloranilin, Toluidin, Xylidin, Aminobenzonitril, Aminobenzoesäure, Dimethylanthranilamid und Diäthylsulfanilamid.

Die gemäss dem erfindungsgemässen Verfahren erhaltenen 1,8-Naphtholactamverbindungen der Formel I können als Zwischenprodukte in der Farbstoffsynthese verwendet werden, z. B. zur Herstellung von Säure- und Dispersionsfarbstoffen, aber auch von kationischen Farbstoffen nach bekannten Verfahren (siehe z. B. EP-A-0 017 621).

Das Verfahren gemäss der vorliegenden Erfindung zeichnet sich gegenüber den bekannten Verfahren zur Herstellung der Naphtholactamverbindungen der Formel I vorallem durch seine Wirtschaftlichkeit aus.

Verbindungen der Formel I mit R = H werden nach bekannten Verfahren z. B. derart erhalten, dass man 1-Naphthylamin mit Phosgen umsetzt und dann den Lactamring nach Friedel-Crafts mit $AlCl_3$ schliesst. Man gelangt auch zur gleichen Verbindung, wenn man 1,8-Naphthalindicarbonsäureanhydrid mit Hydroxylamin und anschliessend mit Tosylchlorid zum N-Tosyloxy-1,8-Naphthalindicarbimid umsetzt und dieses nach Lossen abbaut. Verbindungen der Formel I, worin R nicht H ist, erhält man mittels einer mehrstufigen Synthese über die Verbindung I mit R = H und z. B. nachfolgender Alkylierung ; es handelt sich also durchweg um mehrstufige Verfahren.

Die Umsetzung von Lactonen, die sich von Hydroxysäuren mit aliphatischer Hydroxygruppe ableiten zu den entsprechenden Lactamen oder N-Alkyllactamen, je nachdem, ob man die Reaktion mit Ammoniak oder einem Amin durchführt, ist bereits in der Literatur beschrieben. So z. B. im Theilheimer Bd. 29 ; 201 (1975) die Synthese einer Zwischenstufe des Alkaloids Dendrobin. Danach wird ein 1,7-Hexahydroindanonlacton mit Methylamin zum entsprechenden N-Methyllactam umgesetzt. Die Reaktion wird in Salzsäure durchgeführt. Die Umsetzung von Isocumarin, das sich ebenfalls von einer Hydroxycarbonsäure mit aliphatischer, hier α,β-ungesättigter Hydroxygruppe, ableitet, mit Ammoniak zum Isocarbostyril ist im Houben-Weyl Bd. VI/2, Seite 793 (1963), beschrieben. Daneben findet sich jedoch in der Literatur der Hinweis, dass Lactone, die sich von Hydroxysäuren mit phenolischer Hydroxylgruppe ableiten, mit Ammoniak oder Aminen keine Lactame bilden [Houben-Weyl Bd XI/2, Seite 539 (1958)].

Die nachfolgenden Beispiele veranschaulichen die Erfindung, ohne sie darauf zu limitieren. Teile bedeuten, sofern nichts anderes angegeben ist, Gewichtsteile und die Temperaturen sind in Grad Celsius angegeben.

Beispiel 1

In einem Autoklaven werden 20 ml (ca. 5 Aequivalente) wässriges Ammoniak vorgelegt und 8,5 g 1,8-Naphtholacton (in kristalliner Form) langsam eingetragen. Nach einigen Minuten Rühren werden 20 ml (ca. 2 Aequivalente) $NaHSO_3$-Lösung (40 %ige wässrige Lösung) zugegeben. Anschliessend wird im Autoklaven für 15 Stunden bei 150 °C gerührt (Druck ca. 7,5 bar). Sodann wird mit ca. 100 ml Wasser verdünnt und abgesaugt. Der Rückstand wird mit ca. 200 ml Wasser gewaschen und im Vakuum getrocknet. Man erhält 7,7 g (= 91 % bezogen auf das eingesetzte 1,8-Naphtholacton) der Naphtholactamverbindung der Formel

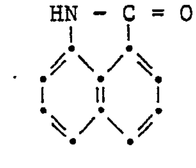
,

welche einen Schmelzpunkt (roh) von 180° aufweist.

Beispiel 2

Man löst 4,1 T 3-Methyl-N-methyl-benzomorpholin in 12,5 T Dichloräthan, gibt nacheinander 4,2 T 1,8-Naphtholactam, erhalten gemäss Beispiel 1, 3 T Phosphorpentoxyd und 10 T Phosphoroxytrichlorid zu und erwärmt auf 80°. Man kondensiert während 1 Stunde bei 81-83°, kühlt auf 25°, tropft langsam 100 T Wasser zu und stellt mit einer wässrigen Lösung von Natriumhydroxyd auf pH ca. 9. Man extrahiert mit 200 T Essigester und destilliert das Lösungsmittel ab. Man erhält so 7,5 T des Farbstoffes der Formel

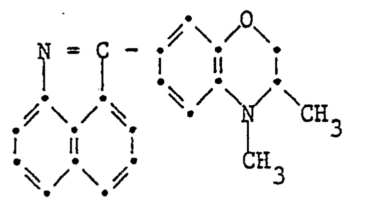

welcher z. B. mit Essigsäure protoniert in Wasser löslich wird. Man erhält mit dieser Lösung eine rotstichig blaue Färbung auf Polyacrylnitrilfasern mit sehr guten Allgemeinechtheiten.

Beispiel 3

Das unsubstituierte 1,8-Naphtholactam lässt sich auch erhalten, indem man die Reaktion wie in Beispiel 1 angegeben durchführt, jedoch kein Bisulfit zusetzt. Das so erhaltene Produkt ist dünnschichtchromatographisch identisch mit gemäss Beispiel 1 erhaltenem 1,8-Naphtholactam.

Beispiel 4

1,8-Naphtholactam lässt sich auch wie folgt herstellen : 8,5 Teile 1,8-Naphtholacton und 19,0 Teile Natriumbisulfit werden in 40 Teilen Methanol vermischt und danach 25 Teile einer gesättigten Lösung von Ammoniak in Methanol zugegeben. Nach 15 Stunden bei 150 °C und 14 bar im Autoklaven erhält man in guter Ausbeute 1,8-Naphtholactam, das mit dem im Beispiel 1 hergestellten Produkt nach dem Dünnschichtchromatogramm wie auch dem Schmelzpunkt, identisch ist.

Beispiele 5 bis 22

Arbeitet man wie in den Beispielen 1, 3 oder 4 beschrieben, setzt jeweils das Naphtholacton aus Spalte 1 der folgenden Tabelle mit dem Amin aus der Spalte 2 um, so erhält man in guter Ausbeute und hoher Qualität das in Spalte 3 aufgeführte Naphtholactam, dessen Schmelzpunkt in Spalte 4 angegeben ist.

(Siehe Schema Seite 5 ff.)

| 1 | | 2 | 3 | 4 |
|---|---|---|---|---|
| 5 | 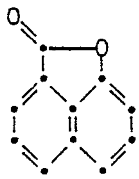 | $NH_2CH_3$ | 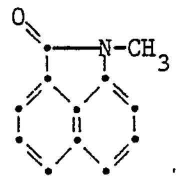 | 74-76°C |
| 6 | do. | $NH_2 \cdot CH_2 \cdot CH_3$ | 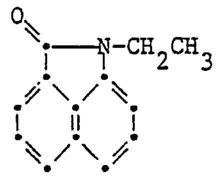 | 69-70°C |
| 7 | do. | $NH_2 \cdot CH_2CH_2CH_3$ | 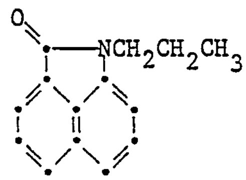 | Oel |
| 8 | do. | $NH_2 \cdot CH_2 \cdot CH_2 \cdot O \cdot CH_3$ | 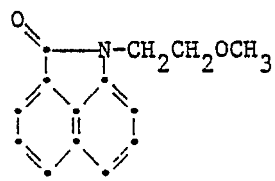 | 65-67°C |
| 9 | do. | $H_2N \cdot CH_2CH_2CN$ | 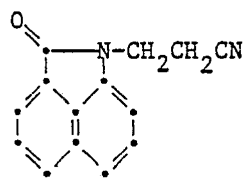 | 125-126°C |
| 10 | do. | 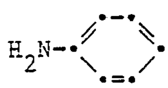 | 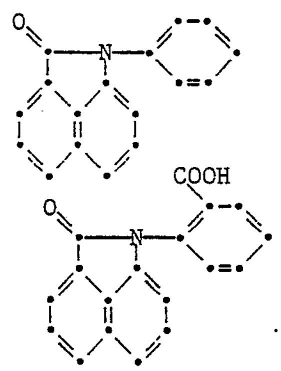 | 99-102°C |
| 11 | do. | 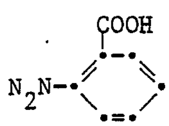 | | 222-223°C |

| 1 | | 2 | 3 | 4 |
|---|---|---|---|---|
| 12 | 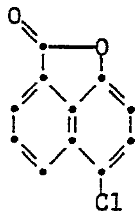 | $NH_2 \cdot CH_3$ | 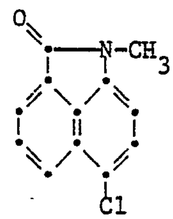 | 131-133°C |
| 13 | 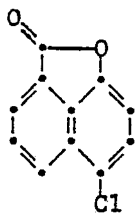 | $NH_2 \cdot CH_2 \cdot CH_3$ | 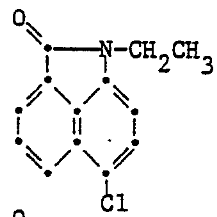 | 152-154°C |
| 14 | do. | $H_2N \cdot CH_2CH_2CN$ | 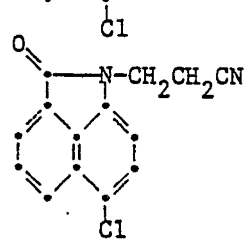 | 170°C |
| 15 | 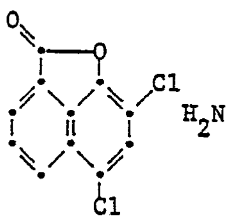 | $H_2N \cdot CH_2CH_2CN$ | 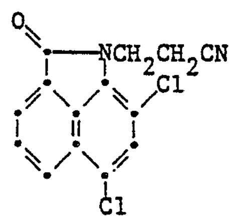 | 150°C |
| 16 | 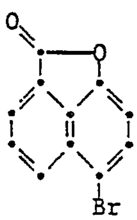 | $NH_3$ | 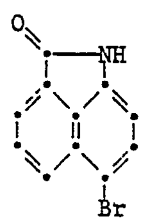 | 251-252°C |
| 17 | do. | $H_2N \cdot CH_3$ | 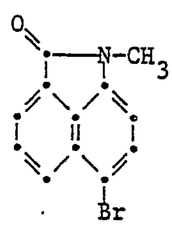 | 131-132°C |

| 1 | 2 | 3 | 4 |
|---|---|---|---|
| 18 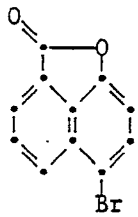 | $H_2N\cdot CH_2CH_3$ | 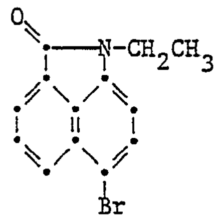 | 103-107°C |
| 19 do. | $H_2N\cdot CH_2CH_2CN$ | 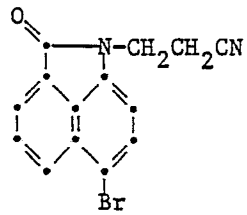 | 200-205°C |
| 20 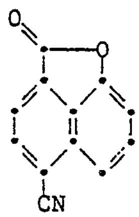 | $H_2N\cdot CH_3$ | 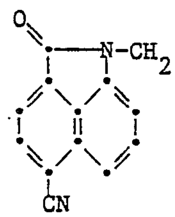 | 204-209°C |
| 21 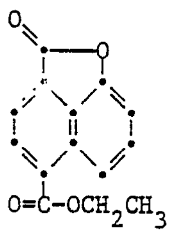 | $H_2N\cdot CH_2CH_3$ | 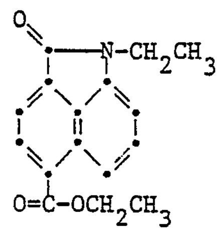 | 80-83°C |
| 22 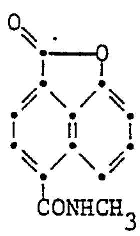 | $H_2N\cdot CH_3$ | 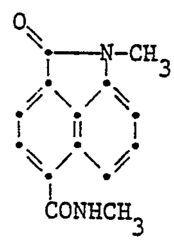 | 206-207°C. |

**Patentansprüche**

1. Verfahren zur Herstellung von 1,8-Naphtholactamverbindungen der Formel I

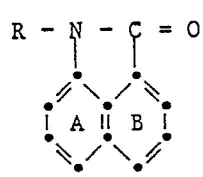

(I)

worin R Wasserstoff, ein gegebenenfalls durch $C_1$-$C_4$-Alkoxy, Halogen, Hydroxy, Cyano, Carboxy, N,N-Dialkyl($C_1$-$C_4$)-carbamoyl oder Phenyl substituierter $C_1$-$C_4$-Alkylrest, ein Cyclohexylrest oder ein gegebenenfalls durch Halogen, $C_1$-$C_4$-Alkyl, $C_1$-$C_4$-Alkoxy, Cyano, Carboxy, N,N-Dialkyl($C_1$-$C_4$)-carbamoyl- oder N,N-Dialkyl($C_1$-$C_4$)-sulfamoyl substituierter Phenylrest ist und die Benzolringe A und/oder B gegebenenfalls durch Halogen, Cyano, Nitro, $C_1$-$C_4$-Alkyl, $C_1$-$C_4$-Alkoxy, $C_1$-$C_4$-Alkylsulfonyl, Phenylsulfonyl, Alkyl-($C_1$-$C_4$)-carbonylamino, Benzoylamino, Carboxyl, $C_1$-$C_4$-Alkoxycarbonyl, Carbamoyl, N-Mono- oder N,N-Dialkyl-($C_1$-$C_4$)-carbamoyl, Sulfamoyl, N-Mono- oder N,N-Dialkyl-($C_1$-$C_4$)-sulfamoyl substituiert sind,

dadurch gekennzeichnet, dass man eine 1,8-Naphtholactonverbindung der Formel II

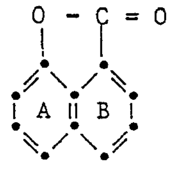

(II)

mit einem Amin der Formel III

$NH_2$—R (III)

gegebenenfalls in Gegenwart von Bisulfit umsetzt.

2. Verfahren gemäss Anspruch 1, dadurch gekennzeichnet, dass man die Umsetzung im wässrigem Medium durchführt.

3. Verfahren gemäss den Ansprüchen 1 und 2, dadurch gekennzeichnet, dass die Umsetzung der Verbindung II mit dem Amin der Formel III in Gegenwart von Bisulfit durchgeführt wird.

4. Verfahren gemäss den Ansprüchen 1 bis 3, dadurch gekennzeichnet, dass man 2 bis 10 Aequivalente des Amins der Formel III und 0,1-5 Aequivalente der Bisulfit-Verbindung, je bezogen auf die 1,8-Naphtholactonverbindung der Formel II, verwendet.

5. Verfahren gemäss Anspruch 4, dadurch gekennzeichnet, dass man 2 bis 5 Aequivalente des Amins der Formel III und 0,5-2,5 Aequivalente der Bisulfit-Verbindung, je bezogen auf die 1,8-Naphtholactonverbindung der Formel II verwendet.

**Claims**

1. A process for the preparation of a 1,8-naphtholactam compound of the formula I

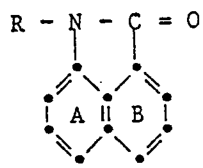

(I)

wherein R is hydrogen, a $C_1$-$C_4$-alkyl group which is unsubstituted or substituted by $C_1$-$C_4$-alkoxy, halogen, hydroxy, cyano, carboxy, N,N-di-($C_1$-$C_4$-alkyl)-carbamoyl or phenyl, or is a cyclohexyl group, or a phenyl group which in unsubstituted or substituted by halogen, $C_1$-$C_4$-alkyl, $C_1$-$C_4$-alkoxy, cyano, carboxy, N,N-di-($C_1$-$C_4$-alkyl)-carbamoyl or N,N-di-($C_1$-$C_4$-alkyl)-sulfamoyl, and the benzene rings A and/or B are unsubstituted or substituted by halogen, cyano, nitro, $C_1$-$C_4$-alkyl, $C_1$-$C_4$-alkoxy, $C_1$-$C_4$-alkylsulfonyl, phenylsulfonyl, $C_1$-$C_4$-alkylcarbonylamino, benzoylamino, carboxy, $C_1$-$C_4$-alkoxycarbonyl, carbamoyl, N-mono- or N,N-di-($C_1$-$C_4$-alkyl)-carbamoyl, sulfamoyl, N-mono- or N,N-di-($C_1$-$C_4$-alkyl)-sulfamoyl, which process comprises reacting a 1,8-naphtholactone compound of the formula II

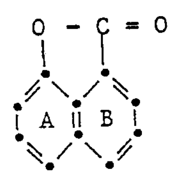

(II)

with an amine of the formula III

$NH_2$—R (III)

in the absence or presence of bisulfite.

2. A process according to claim 1, wherein the reaction is performed in an aqueous medium.

3. A process according to either of claims 1 or 2, wherein the reaction of the compound II with the amine of the formula III is performed in the presence of bisulfite.

4. A process according to any one of claims 1 to 3, which comprises the use of 2 to 10 equivalents of the amine of the formula III and 0.1 to 5 equivalents of the bisulfite compound, based in each case on the 1,8-naphtholactone compound of the formula II.

5. A process according to claim 4, which comprises the use of 2 to 5 equivalents of the amine of the formula III and 0.5 to 2.5 equivalents of the bisulfite compound, based in each case on the 1,8-naphtholactone compound of the formula II.

**Revendications**

1. Procédé pour la préparation de composés 1,8-naphtolactames de formule I

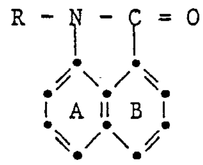

(I)

dans laquelle R est l'hydrogène, un radical alkyle en $C_1$-$C_4$ éventuellement substitué par des radicaux alcoxy en $C_1$-$C_4$, halogéno, hydroxy, cyano, carboxy, N,N-di-(alkyl en $C_1$-$C_4$)-carbamoyle ou phényle ; un radical cyclohexyle ou un radical phényle éventuellement substitué par des radicaux halogènes, alkyle en $C_1$-$C_4$, alcoxy en $C_1$-$C_4$, cyano, carboxy, N,N-di-(alkyl en $C_1$-$C_4$)-carbamoyle ou N,N-di-(alkyl en $C_1$-$C_4$)-sulfamoyle ; et les noyaux benzéniques A et/ou B sont éventuellement substitués par des radicaux halogéno, cyano, nitro, alkyle en $C_1$-$C_4$, alcoxy en $C_1$-$C_4$, (alkyl en $C_1$-$C_4$)-sulfonyle, phénylsulfonyle, (alkyle en $C_1$-$C_4$)-carbonylamino, benzoylamino, carboxyle, (alcoxy en $C_1$-$C_4$)-carbonyle, carbamoyle, N-mono- ou N,N-di-(alkyl en $C_1$-$C_4$)-carbamoyle, sulfamoyle, N-mono ou N,N-di-(alkyl en $C_1$-$C_4$)-sulfamoyle, caractérisé en ce qu'on fait réagir un composé 1,8-naphtolactone de formule II

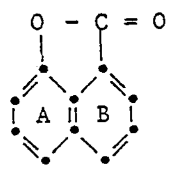

(II)

sur une amine de formule III

$$NH_2—R \qquad \text{(III)}$$

éventuellement en présence d'un bisulfite.

2. Procédé selon la revendication 1, caractérisé en ce qu'on procède à la réaction en milieu aqueux.

3. Procédé selon les revendications 1 et 2, caractérisé en ce que la réaction du composé II sur l'amine de formule III est mise en œuvre en présence d'un bisulfite.

4. Procédé selon les revendications 1 à 3, caractérisé en ce qu'on utilise 2 à 10 équivalents de l'amine de formule III et 0,1 à 5 équivalents du composé bisulfite, chaque fois par rapport au composé 1,8-naphtolactone de formule II.

5. Procédé selon la revendication 4, caractérisé en ce qu'on utilise 2 à 5 équivalents de l'amine de formule III et 0,5 à 2,5 équivalents du composé bisulfite, chaque fois par rapport au composé 1,8-naphtolactone de formule II.